Europäisches Patentamt

(19) European Patent Office

Office européen des brevets

(11) Publication number: **0 079 934**
**B1**

(12) **EUROPEAN PATENT SPECIFICATION**

(45) Date of publication of patent specification: **29.04.87**

(51) Int. Cl.⁴: **C 07 K 7/06**, C 07 K 7/08, A 61 K 37/00

(21) Application number: **82901897.7**

(22) Date of filing: **11.05.82**

(86) International application number:
**PCT/US82/00619**

(87) International publication number:
**WO 82/04250 09.12.82 Gazette 82/29**

(54) **ANTIGENIC LINEAR PEPTIDE COMPOUNDS.**

(30) Priority: **26.05.81 US 267021**
**26.06.81 US 277623**
**06.07.81 US 280295**

(43) Date of publication of application:
**01.06.83 Bulletin 83/22**

(45) Publication of the grant of the patent:
**29.04.87 Bulletin 87/18**

(84) Designated Contracting States:
**AT DE FR GB LU NL SE**

(56) References cited:
**US-A-4 310 456**

**Chemical Abstract, vol. 78, 1973, page 41298g, B. Johnson**

**Chemical Abstracts, vol. 95, 1981, page 128346w, R. Hensel**

**The file contains technical information submitted after the application was filed and not included in this specification**

(73) Proprietor: **NORTHWESTERN UNIVERSITY**
**Rebecca Crown Center**
**Evanston Illinois 60201 (US)**

(72) Inventor: **GOLDBERG, Erwin**
**2732 Hampton Parkway**
**Evanston, IL 60201 (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22 (DE)**

(56) References cited:

**Journal of Biological Chemistry, vol. 254, no. 16, issued 25 Aug. 1979, pages 7621-7623, Musick et al.**

**Molecular Immunology, vol. 19/12, (1984), 1579-1585**

# 0 079 934

**Description**

Mammalian spermatozoa have been known to be antigenic for many years. More recently, it has been demonstrated that mammalian sperm contain an antigenic enzyme, which is known as the $C_4$ isozyme of lactate dehydrogenase (LDH—X, LDH—$C_4$). LDH—$C_4$ has been isolated in pure crystalline form from mouse testes. Goldberg (1972) *J. Biol. Chem.* 247: 2044—2048. The enzyme has a molecular weight of 140,000 and is composed of four identical C subunits. The amino acid sequence and three-dimensional structure of LDH—$C_4$ has been studied and partially determined by a number of investigators. See Musick et al (1976) *J. Mol. Biol.* 104: 659—668; and Wheat et al (1977) *Biochem & Biphys. Res. Comm.,* 74, No. 3: 1066—1077. Wheat et al determined the sequence of the essential thiol peptide from amino acid 159 to 171, and found this to be nearly identical to essential thiol peptides from other vertebrate LDH isozymes.

In 1974, Dr. Erwin Goldberg reviewed the effects of immunization with LDH—X (LDH—$C_4$) on fertility, and advanced the possibility that "by using a defined macromolecular constituent of sperm it becomes possible to elucidate its primary structure in terms of amino acid sequence, to map specifically the antigenic determinant(s) responsible for inducing infertility, and then to construct synthetic peptides containing these determinants. Possessing the capability for synthesizing a molecule with such properties, makes the immunological approach to fertility control feasible." Karolinska Symposia on Research Methods in Reproductive Endocrinology, 7th Symposia: Immunological Approaches to Fertility Control, Geneva, 1974 202—222. However, such synthetic antigenic peptides remained a goal and not achievement, although their theoretical desirability has been recognized. In 1979, Dr. Erwin Goldberg summarized the state of the art as follows:

"In conclusion, and on a practical basis, immunotherapy for birth control requires more than effectiveness, specificity, reversibility, and absence of systemic side reaction. Rather large amounts of the antigen must be available in unequivocally pure form. This condition probably cannot be met by a natural product enzyme antigen from sperm or testes. Rather, contraceptive technology requires a synthesizable peptide fragment retaining antigenicity and provoking a response which impairs fertility. Completion of the structural analysis of LDH—$C_4$ should allow mapping of antigenic determinants and synthesis of such peptides for use in a new contraceptive technology". *"Recent advances in Reproduction and Regulation of Fertility,"* G. P. Talwar, editor, Elsevier/North Holland Biomedical Press (1979).

It has now been discovered that highly antigenic peptides can be prepared by synthesizing linear sequences of amino acids which sequences are believed to correspond to amino acid sequence present in the natural enzyme LDH—$C_4$. These sequences include: glutamic acid-glutamine-leucine-isoleucine-glutamine-asparagine-leucine-valine-proline-glutamic acid-aspartic acid-lysine, which is believed to correspond to the LDH—$C_4$ amino acids 5 to 16; isoleucine-serine-glycine-phenylalanine-proline-valine-glycine-arginine, which is believed to correspond to the LDH—$C_4$ amino acids 152 to 159; and serine-leucine-asparagine-proline-alanine-isoleucine-glycine-threonine-aspartic acid-lysine, which is believed to correspond with the LDH—$C_4$ amino acids 211 to 220. Sequence 152 to 159 corresponds with the published tentative mapping of LDH—$C_4$, but sequences 5 to 16 and 211 to 220 do not. See Musick et al (August 25, 1979), *J. Biol. Chem.,* 254, No. 16: 7621—7623.

Compounds embodying the antigenic sequences identified above include the following specific compounds:

    (a) N-Glu-Gln-Leu-Ile-Gln-Asn-Leu-Val-Pro-Glu-Asp-Lys-C,
    (b) N-Glu-Gln-Leu-Ile-Gln-Asn-Leu-Val-Pro-Glu-Asp-Lys-Leu-C,
    (c) N-Glu-Gln-Leu-Ile-Gln-Asn-Leu-Val-Pro-Glu-Asp-Lys-Leu-Ser-C,
    (d) N-Glu-Gln-Leu-Ile-Gln-Asn-Leu-Val-Pro-Glu-Asp-Lys-Leu-Ser-Arg-C,
    (e) N-Cys-Glu-Gln-Leu-Ile-Gln-Asn-Leu-Val-Pro-Glu-Asp-Lys-C,
    (f) N-Cys-Glu-Gln-Leu-Ile-Gln-Asn-Leu-Val-Pro-Glu-Asp-Lys-Leu-C,
    (g) N-Cys-Glu-Gln-Leu-Ile-Gln-Asn-Leu-Val-Pro-Glu-Asp-Lys-Leu-Ser-C,
    (h) N-Cys-Glu-Gln-Leu-Ile-Gln-Asn-Leu-Val-Pro-Glu-Asp-Lys-Leu-Ser-Arg-C,
    (i) N-Ile-Ser-Gly-Phe-Pro-Val-Gly-Arg-C,
    (j) N-Ile-Ser-Gly-Phe-Pro-Val-Gly-Arg-Val-C,
    (k) N-Gly-Ile-Ser-Gly-Phe-Pro-Val-Gly-Arg-C,
    (l) N-Gly-Ile-Ser-Gly-Phe-Pro-Val-Gly-Arg-Val-C,
    (m) N-Ser-Leu-Asn-Pro-Ala-Ile-Gly-Thr-Asp-Lys-C,
    (n) N-Cys-Ser-Leu-Asn-Pro-Ala-Ile-Gly-Thr-Asp-Lys-C.

In the foregoing formulas, the letter "N" designates the N-terminal amino. acid, while the letter "C" designates the C-terminal amino acid. Gly represents glycine, and Glu, Gln, Leu, Ile, Asn, Val, Pro, Asp, Lys, Ser, Arg, Cys, Phe, Ala, and Thr, respectively represent the L-amino acid forms of glutamic acid, glutamine, leucine, isoleucine, asparagine, valine, proline, aspartic acid, lysine, serine, arginine, cysteine, phenylalanine, alanine, and threonine. As will be noted compounds (a) to (h) include the 12 amino acid sequence, starting with glutamine acid and ending with lysine which is believed to correspond to amino acids 5 to 16 of LDH—$C_4$. The additional amino acids of compounds (b), (c), and (d) are believed to correspond to the next amino acids of LDH—$C_4$, that is numbers 16, 17, and 18. Compound (d) therefore

2

0 079 934

would contain the amino acids of the sequence 5 to 18. The compounds (e) to (h) include the same antigenic sequences, respectively, of compounds (a) to (d), but cysteine has been added at the N-terminal end to facilitate the coupling of these compounds to proteins in preparing antigenic vaccines. Similarly, compound (n) contains the same antigenic sequence of compound (m) except that cysteine has been added at the N-terminal end for the same purpose.

Compounds (i) to (e) all include the 8 amino acid sequence beginning with isoleucine and ending with valine. For compound (j), valine is added at the C-terminal end to act as a spacer amino acid and thereby enhance immunogenicity. This will move the antigenic binding site further from the free carboxyl group. Alternatively or additionally glycine may be added to the N-terminal end to serve as a spacer for improving immunogenicity, as provided, respectively, in compounds (k) and (l). Other amino acids can similarly be used as spacers at the terminal ends of the antigenic sequences described above. Further, as will be apparent to those skilled in the art of immunology, other C- and N-terminal amino acids can be substituted while retaining or enhancing antigenicity. The preferred compounds however, are those set out above. It is believed that compounds (a) to (h) are particularly effective.

The peptide compounds of the present invention can be synthesized from their constituent amino acids. For example, the synthesis can be carried out by the Merrifield solid phase method, as described in *J.A.C.S.* 85: 2149—2154 (1963). This solid phase method for synthesizing sequences of amino acids is also described in Stewart and Young, *Solid Phase Peptide Synthesis* (W. H. Freeman and Co. San Francisco, 1969), pages 1—4. In this procedure, the C-terminal amino acid, such as lysine for compounds (a) and (e) or leucine for compounds (b) and (f), is attached to chloromethylated polystyrene-divinylbenzene copolymer beads. Each subsequent amino acid, with suitable protecting group, is then added sequentially to the growing chain. For example, as described in the Merrifield article, the protective group may be a carbobenzoxy group. By the procedure of coupling, deprotection, and coupling of the next amino acid, the desired amino acid sequence and chain length can be produced. As a final step, the protective group is removed from the N-terminal amino acid, and the C-terminal amino acid is cleaved from the resin, using a suitable reagent, such as trifluoroacetic acid and hydrogen bromide. Compounds (a) to (n) as described above, are prepared by this synthesis procedure for use in reducing the fertility of mammals.

To utilize antigenic peptides of this invention in the form of fertility reducing vaccines, the peptide is conjugated to a carrier molecule, which is preferably a protein which itself elicits an antigenic response and which can be safely administered. For example, the peptide can be coupled to tetanus toxoid for administration by intramuscular injection. For example, a mixture of 1 µMole tetanus toxoid, 60 µMoles antigenic peptide, and 18 millimoles 1 - ethyl - 3 - (3 dimethyl aminopropyl) carbodiimide hydrochloride reacted in water (pH 6) for 12 hours at room temperature and 24 hours at 4° gives a product containing 3.5 moles of peptide/mole of tetanus toxoid. Excess reactants can be removed by dialysis or gel filtration. See Pique et al, *Immunochemistry,* 15: 55—60 (1978). Alternatively, the peptide may be coupled using bisdiazotized benzidine (Bassiri et al, *Endocrinology*, 90: 722 (1972)) or glutaraldehyde.

For intramuscular injection, the coupled peptide may be suspended in a sterile isotonic saline solution, or other conventional vehicle, and, if desired, an adjuvant may be included. A preferred use of such a vaccine is for administration to human females. Anti-bodies will be formed, which will appear in the oviduct fluids and thereby achieve a significant reduction in fertility. For this purpose, the amount to be administered will range from about 1 to 10 milligrams (mg) of the antigenic peptide.

The peptide compounds of this invention and their method of preparation are further illustrated by the following examples.

Example I

Preparation of linear peptide Cys-Glu-Gln-Leu-Ile-Gln-Asn-Leu-Val-Pro-Glu-Asp-Lys

Synthesis of the above peptide, referred to herein as compound (e), can be carried out employing solid phase techniques now well known in the art. In a preferred procedure amino protected lysine, representing the —COOH terminal group of the above peptide, is coupled to a conventional solid phase peptide synthesis resin such as chloromethyl polystyrene cross-linked with 1 to 2% divinyl benzene. The amino protecting group is then selectively removed utilizing a suitable reagent whose nature will depend on the protecting group used. In the preferred embodiment the t-butyloxycarbonyl (Boc) group is utilized for amino group protection and 40% trifluoroacetic acid in methylene chloride is the selective deprotecting agent.

After deprotection, the lysine-resin is treated with protected aspartic acid, preferably N-Boc-O-Benzyl aspartic acid, and dicyclohexylcarbodiimide in a manner known per se as to form a peptide bond between the free amino group of the lysine residue and the carboxyl group of protected aspartic acid.

The cycle of deprotection and coupling with amino acid derivatives and dicyclohexylcarbodiimide is then repeated with the remaining amino acids in the sequence order of the above peptide. Some of the amino acids required side-chain blocking groups besides the alpha-amino protection. Such amino acids and the blocking groups are as follows:

Cys(MBzl), Glu(oBzl), Asp(oBsl), Lys(Cl-z) where oBzl is benzyl, Cl-z is o-chlorobenzyloxycarbonyl and MBzl is methoxybenzyl.

Completion of the synthesis provided the following peptide coupled to the styrenedivinylbenzene copolymer resin:

3

TFA-Cys(MBzl)Glu(oBzl)-Gln-Leu-Ile-Gln-Asn-Leu-Val-Pro-Glu(oBzl)-Asp(oBzl)-Lys(Cl-z)-Res

Decoupling of the peptide from the resin is accomplished by treatment with liquid hydrogen fluoride with concomitant cleavage of all protecting groups to produce the desired peptide.

Solid phase synthesis of N-Boc-Lys(Cl-z) resin

Attachment of N-Boc-Lys(Cl-z) to chloromethyl resin was performed by the cesium salt method. A sample of chloromethyl resin (200 g) containing 0.74 mmol chloride per gram is treated with the cesium salt of Boc-Lys-(Cl-z) resulting from the neutralization of Boc-lysine with cesium carbonate. About 73.8 grams of Boc-Lysine is dissolved in 80% methanol and 20% water and adjusted to pH 7.0 with about 29 grams of cesium carbonate. The resulting solution is dried on a rotary evaporator, then dried three more times after three additions of 100 milliliters of xylene. To the cesium salt of Boc-Lys(Cl-z) was added 200 grams of chloromethyl resin as above and sufficient 1-Methyl-2-Pyrrolidinone to make about 1.90 liters total volume. The resulting mixture is stirred at 55°C for 48 hours. The resin was then washed extensively with methanol, then water, then again with methanol. The resin was air dried, then dried under vacuum. After cleavage of lysine from resin with HF, amino acid analysis gave one peak corresponding to 0.36 mmol/gm.

A sample of the resin just described (6.0 g) was submitted to the following synthesis schedule: (1) Wash with three 100 ml portions of methylene chloride; (2) removal of the Boc group with 40% TFA in methylene chloride for a one minute wash and for a 20 minute reaction time; (3) wash with three 100 ml portions of methylene chloride; (4) wash with two 100 ml portions of isopropanol; (5) wash with three 100 ml portions of methylene chloride; (6) a one minute wash and ten minutes neutralization with 100 ml portions of 10% triethylamine in methylene chloride; (7) wash with three portions of 100 ml of methylene chloride; (8) add 2.5 equivalents (7.2 mmol) of Boc amino acid and 2.5 equivalents (7.2 mmol) of dicyclohexylcarbodiimide in methylene chloride and shake for 2 hours; (9) wash with three 100 ml portions of methylene chloride; (10) wash with two 100 ml portions of isopropanol; (11) wash with three 100 ml portions of methylene chloride. The above cycle was repeated for the following N-protected amino acids:

Boc-Arg(Tos)
Boc-Gly
Boc-Val
Boc-Pro
Boc-Phe
Boc-Gly
Boc-Ser(oBzl)
Boc-Ile
Boc-Gly.

The protected peptide resin was submitted to deprotection to give the TFA salt of the deprotected peptide resin. The dried resin was stirred in the presence of 6 ml of anisole and 60 ml of liquid HF at 0°C for 1 hour. The HF was removed by vacuum and the oily residue was washed with two 50 ml portions of ethyl ether. The peptide was extracted from the resin by three 50 ml portions of 1 molar acetic acid and the combined filtrates were lyophilized to give 8.66 grams of crude peptide. A sample (1.5 g) was purified by 250 transfers in a counter-current distribution apparatus and recovery of the appropriate fractions gave 0.994 g of highly purified peptide. The solvent system for the above fractionation was butanol:acetic acid:water at 4:1:5 ratios.

Amino acid analysis of the peptide after acid hydrolysis gave: $Arg_{1.00}$, $Ser_{1.03}$, $Pro_{1.01}$, $Gly_{3.05}$, $Val_{2.08}$, $Ile_{0.98}$, $Phe_{1.03}$. The peptide gave single spots in two systems with silica gel thin layer chromatography. These systems were the upper layer of butanol:acetic acid:water of 4:1:5 and the system chloroform:methanol:water of 60:30:5. The $R_f$ values for the peptide in the two systems was 0.20 and 0.32 respectively. On paper electrophoresis, the peptide gave a single spot migrating to the cathode with a $R_f$ compared to picric acid of 0.54. Electrophoresis conditions were Whatman 3MM paper with a pyridine-acetate buffer at pH 5.6.

Compounds (a) to (d) and (f) to (h) containing the same antigenic sequence as compound (e) are prepared in the same manner and characterized in the same way.

Example II
Preparation of linear peptide Gly-Ile-Ser-Gly-Phe-Pro-Val-Gly-Arg-Val

Synthesis of the above peptide referred to herein as compound (1), can be carried out employing solid phase techniques now well known in the art. In a preferred procedure amino protected valine, representing the —COOH terminal group of the above peptide, is coupled to a conventional solid phase peptide synthesis resin such as chloromethyl polystyrene cross-linked with 1 to 2% divinyl benzene. The amino protecting group is then selectively removed utilizing a suitable reagent whose nature will depend on the protecting group used. In the preferred embodiment the t-butyloxycarbonyl (Boc) group is utilized for amino group protection and 40% trifluoroacetic acid in methylene chloride is the selective deprotecting agent.

After deprotection, the valine-resin is treated with protected arginine, preferably N-Boc-$N^6$-Tosyl Arginine, and dicyclohexylcarbodiimide in a manner known per se as to form a peptide bond between the free amino group of the valine residue and the carboxyl group of protected arginine.

The cycle of deprotection and coupling with amino acid derivatives and dicyclohexylcarbodiimide is then repeated with the remaining amino acids in the sequence order of the above peptide. Some of the amino acids required side-chain blocking groups besides the alpha-amino protection. Such amino acids and the blocking groups are as follows:

Ser(oBzl) and Arg(Tos) where oBzl is benzyl and Tos is guanidino-p-Toluenesulfonyl.

Completion of the synthesis provided the following decapeptide coupled to the styrenedivinylbenzene copolymer resin:

TFA Gly-Ile-Ser-(oBzl)-Gly-Phe-Pro-Val-Gly-Arg-(Tos)-Val-Resin

Decoupling of the peptide from the resin is accomplished by treatment with liquid hydrogen fluoride with concomitant cleavage of all protecting groups to produce the desired peptide.

Solid phase synthesis of Gly-Ile-Ser-Gly-Phe-Pro-Val-Gly-Arg-Val
N-Boc-Val-Resin

Attachment of N-Boc-Valine to chloromethyl resin was performed by the cesium salt method. A sample of chloromethyl resin (200 g) containing 0.74 mmol chloride per gram is treated with the cesium salt of Boc-Valine resulting from the neutralization of Boc-Valine with cesium cabronate. About 38.6 grams of Boc-Valine is dissolved in 80% methanol and 20% water and adjusted to pH 7.0 with about 29 grams of cesium carbonate. The resulting solution is dried on a rotary evaporator, then dried three more times after three additions of 100 milliliters of xylene. To the cesium salt of Boc-Valine was added 200 grams of chloromethyl resin as above and sufficient 1 - Methyl - 2 - Pyrrolidinone to make about 1.90 liters total volume. The resulting mixture is stirred at 55°C for 48 hours. The resin was then washed extensively with methanol, then water, then again with methanol. The resin was air dried, then dried under vacuum. After cleavage of valine from the resin with HF, amino acid analysis gave one peak corresponding to 0.48 mmol/gm.

A sample of the resin just described (6.0 g) was submitted to the following synthesis schedule: (1) wash with three 100 ml portions of methylene chloride; (2) removal of the Boc group with 40% TFA in methylene chloride for a one minute wash and for a 20 minute reaction time; (3) wash with three 100 ml portions of methylene chloride; (4) wash with two 100 ml portions of isopropanol; (5) wash with three 100 ml portions of methylene chloride; (6) a one minute wash and ten minutes neutralization with 100 ml portions of 10% triethylamine in methylene chloride; (7) wash with three portions of 100 ml of methylene chloride; (8) add 2.5 equivalents (7.2 mmol) of Boc amino acid and 2.5 equivalents (7.2 mmol) of dicyclohexylcarbodiimide in methylene chloride and shake for 2 hours; (9) wash with three 100 ml portions of methylene chloride; (10) wash with two 100 ml portions of isopropanol; (11) wash with three 100 ml portions of methylene chloride. The above cycle was repeated for the following N-protected amino acids:

| | |
|---|---|
| Boc-Asp(oBzl) | Boc-Gln |
| Boc-Glu(oBzl) | Boc-Ile |
| Boc-Pro | Boc-Leu |
| Boc-Val | Boc-Gln |
| Boc-Leu | Boc-Glu(oBzl) |
| Boc-Asn | Boc-Cys(MBzl) |

The protected peptide resin was submitted to deprotection to give the TFA salt of the deprotected peptide resin. The dried resin (5.88 g) was stirred in the presence of 6 ml of anisole and 60 ml of liquid HF at 0°C for 1 hour. The HF was removed by vacuum and the oily residue was washed with two 50 ml portions of ethyl ether. The peptide was extracted from the resin by three 50 ml portions of 1 molar acetic acid and the combined filtrates were lyophilized to give 2.27 grams of crude peptide. This was purified by 250 transfers in a counter-current distribution apparatus. The solvent system for the above fractionation was butanol:acetic acid:water at 4:1:5 ratios.

Somewhat purified fractions from the countercurrent distribution apparatus were further purified by column chromatography on diethylaminoethylcellulose with a linear gradient of 0.01 to 0.5 molar ammonium bicarbonate to give 356 mg of pure peptide.

Amino acid analysis of the pure peptide after acid hydrolysis gave: $Lys_{1.02}$, $ammonia_{2.87}$, $Asp_{2.16}$, $Glu_{3.82}$, $Pro_{0.96}$, $Cys_{0.85}$, $Val_{0.97}$, $Ile_{0.79}$, $Leu_{1.85}$. This peptide gave a single spot with a Rf of 0.89 on cellulose thin layer chromatography with a solvent system of butanol:ethyl acetate:acetic acid:water of 1:1:1:1.

Compounds (i) to (k) containing the same antigenic sequence as compound (I) are prepared in the same manner and characterized in the same way.

Example III
Preparation of linear peptide Cys-Ser-Leu-Asn-Pro-Ala-Ile-Gly-Thr-Asp-Lys

Synthesis of the above peptide, referred to herein as compound (n), can be carried out employing solid phase techniques now well known in the art. In a preferred procedure amino protected lysine, representing the —COOH terminal group of the above peptide, is coupled to a conventional solid phase peptide synthesis resin such as chloromethyl polystyrene crosslinked with 1 to 2% divinyl benzene. The amino protecting group is then selectively removed utilizing a suitable reagent whose nature will depend on the protecting group used. In the preferred embodiment the t-butyloxycarbonyl (Boc) group is utilized for amino group protection and 40% trifluoroacetic acid in methylene chloride is the selective deprotecting agent.

After deprotection, the lysine resin is treated with protected aspartic acid, preferably N-Boc-O-Benzyl aspartic acid, and dicyclohexylcarbodiimide in a manner known per se as to form a peptide bond between the free amino group of the lysine residue and the carboxyl group of protected aspartic acid.

The cycle of deprotection and coupling with amino acid derivatives and dicyclohexylcarbodiimide is then repeated with the remaining amino acids in the sequence order of the above peptide. Some of the amino acids required side-chain blocking groups besides the alpha-amino protection. Such amino acids and the blocking groups are as follows:

Cys(MBzl)
Ser-(oBzl) Thr(oBzl) Asp(oBzl) Lys(Cl-z)

where oBzl is benzyl and MBzl is p-methoxybenzyl and Cl-z is o-chlorobenzyloxycarbonyl.

Completion of the synthesis provided the following decapeptide coupled to the styrenedivinylbenzene copolymer resin:

TFA-Cys(MBzl)Ser-(oBzl)-Leu-Asn-Pro-Ala-Ile-Gly-Thr(oBzl)-Asp(oBzl)-Lys(Cl-z)-Resin

Decoupling of the peptide from the resin is accomplished by treatment with liquid hydrogen fluoride with concomitant cleavage of all protecting groups to produce the desired peptide.

Solid phase synthesis of Cys(MBzl)-Ser(oBzl)-Leu-Asn-Pro-Ala-Ile-Gly-Thr(oBzl)Asp(oBzl)-Lys(Cl-z) resin

Attachment of N-Boc-Lys(Cl-z) to chloromethyl resin was performed by the cesium salt method. A sample of chloromethyl resin (200 g) containing 0.74 mmol chloride per gram is treated with the cesium salt of Boc-Lysine resulting from the neutralization of N-Boc-Lys(Cl-z) with cesium carbonate. About 73.8 grams of N-Boc-Lys(Cl-z) is dissolved in 80% methanol and 20% water and adjusted to pH 7.0 with about 29 grams of cesium carbonate. The resulting solution is dried on a rotary evaporator, then dried three more times after three additions of 100 milliliters of xylene. To the cesium salt of N-Boc-Lys(Cl-z) was added 200 grams of chloromethyl resin as above and sufficient 1 - Methyl - 2 - Pyrrolidinone to make about 1.90 liters total volume. The resulting mixture is stirred at 55°C for 48 hours. The resin was then washed extensively with methanol, then water, then again with methanol. The resin was air dried, then dried under vacuum. After cleavage of lysine from the resin with HF, amino acid analysis gave one peak corresponding to 0.36 mmol/gm.

A sample of the resin just described (5.5 g) was submitted to the following synthesis schedule: (1) wash with three 100 ml portions of methylene chloride; (2) removal of the Boc group with 40% TFA in methylene chloride for a one minute wash and for a 20 minute reaction time; (3) wash with three 100 ml portions of methylene chloride; (4) wash with two 100 ml portions of isopropanol; (5) wash with three 100 ml portions of methylene chloride; (6) a one minute wash and ten minutes neutralization with 100 ml portions of 10% triethylamine in methylene chloride; (7) wash with three portions of 100 ml of methylene chloride; (8) add 2.5 equivalents (7.2 mmol) of Boc amino acid and 2.5 equivalents (7.2 mmol) of dicyclohexylcarbodiimide in methylene chloride and shake for 2 hours; (9) wash with three 100 ml portions of methylene chloride; (10) wash with two 100 ml portions of isopropanol; (11) wash with three 100 ml portions of methylene chloride. The above cycle was repeated for the following N-protected amino acids:

Boc-Asp(oBzl)    Boc-Leu
Boc-Thr(oBzl)    Boc-Ser(oBzl)
Boc-Gly    Boc-Cys(MBzl)
Boc-Ile
Boc-Ala
Boc-Pro
Boc-Asn

The protected peptide resin was submitted to deprotection to give the TFA salt of the deprotected peptide resin. A portion of the dried resin (6 g) was stirred in the presence of 6 ml of anisole and 60 ml of liquid HF at 0°C for 1 hour. The HF was removed by vacuum and the oily residue was washed with two 50 ml portions of ethyl ether. The peptide was extracted from the resin by three 50 ml portions of 1 molar acetic acid and the combined filtrates were lyophilized.

The peptide was dimerized by stirring a solution in water at pH 9.0 for three days in the presence of air. It was then lyophilized and subjected to counter-current distribution in the system.

0.1% Acetic acid:Butanol:pyridine of 11:5:3. The purest fractions were further purified by column chromatography on carboxymethylcellulose using a gradient of ammonium bicarbonate from 0.01 molar to 0.2 molar. The purest fractions were subjected to column chromatography on diethylaminoethyl cellulose, using an ammonium bicarbonate gradient from 0.01 molar to 0.05 molar. The pure peptide was lyophilized to give 77 mg.

Amino acid analysis of the peptide after acid hydrolysis gave: Lysine$_{1.14}$, ammonia$_{0.86}$, aspartic acid$_{1.19}$, threonine$_{1.12}$, serine$_{0.96}$, proline$_{1.0}$, glycine$_{1.00}$, Alanine$_{1.07}$, cysteine$_{0.98}$, Isoleucine$_{0.86}$, and leucine$_{0.92}$. Thin layer chromatography on cellulose in butanol:ethyl acetate:acetic acid:water of 1:1:1:1 gave an R$_f$ of 0.64 with a minor upper spot. When thin layer chromatography on cellulose with butanol:pyridine:acetic acid:water of 15:10:3:12 was used, the R$_f$ was 0.45, also with a minor upper spot. Paper electrophoresis on Whatman 3MM paper with pyridine acetate buffer at pH 6.4 showed a single spot at the neutral position, R$_f$ 0.20 with reference to picric acid.

Compound (m) containing the same antigenic sequence of compound (n) is prepared in the same manner and characterized in the same way.

**Claims**

1. The antigenic peptide compounds arranged in a sequence from N-terminal to C-terminal amino acids selected from the class consisting of:

(a) Glu-Gln-Leu-Ile-Gln-Asn-Leu-Val-Pro-Glu-Asp-Lys
(b) Glu-Gln-Leu-Ile-Gln-Asn-Leu-Val-Pro-Glu-Asp-Lys-Leu
(c) Glu-Gln-Leu-Ile-Gln-Asn-Leu-Val-Pro-Glu-Asp-Lys-Leu-Ser
(d) Glu-Gln-Leu-Ile-Gln-Asn-Leu-Val-Pro-Glu-Asp-Lys-Leu-Ser-Arg
(e) Cys-Glu-Gln-Leu-Ile-Gln-Asn-Leu-Val-Pro-Glu-Asp-Lys
(f) Cys-Glu-Gln-Leu-Ile-Gln-Asn-Leu-Val-Pro-Glu-Asp-Lys-Leu
(g) Cys-Glu-Gln-Leu-Ile-Gln-Asn-Leu-Val-Pro-Glu-Asp-Lys-Leu-Ser
(h) Cys-Glu-Gln-Leu-Ile-Gln-Asn-Leu-Val-Pro-Glu-Asp-Lys-Leu-Ser-Arg
(i) Ile-Ser-Gly-Phe-Pro-Val-Gly-Arg,
(j) Ile-Ser-Gly-Phe-Pro-Val-Gly-Arg-Val,
(k) Gly-Ile-Ser-Gly-Phe-Pro-Val-Gly-Arg,
(l) Gly-Ile-Ser-Gly-Phe-Pro-Val-Gly-Arg-Val,
(m) Ser-Leu-Asn-Pro-Ala-Ile-Gly-Thr-Asp-Lys, and
(n) Cys-Ser-Leu-Asn-Pro-Ala-Ile-Gly-Thr-Asp-Lys,

wherein Gly represents glycine, and Glu, Gln, Leu, Ile, Asn, Val, Pro, Asp, Lys, Ser, Arg, Cys, Phe, Ala, and Thr, respectively represent the L-amino acid forms of glutamic acid, glutamine, leucine, isoleucine, asparagine, valine, proline, aspartic acid, lysine, serine, arginine, cysteine, phenylalanine, alanine, and threonine.

2. The peptide compounds (a) to (h) of claim 1 which include the 12 amino acid sequence of (a).
3. The peptide compound (e) of claim 1.
4. The peptide compounds (i) to (l) of claim 1 which include the 8 amino acid sequence of (i).
5. The peptide compound (l) of claim 1.
6. The peptide compounds (m) and (n) of claim 1.
7. The peptide compound (n) of claim 1.
8. A method for preparing the antigenic peptide compounds arranged in a sequence from N-terminal to C-terminal amino acids selected from the class consisting of:

(a) Glu-Gln-Leu-Ile-Gln-Asn-Leu-Val-Pro-Glu-Asp-Lys
(b) Glu-Gln-Leu-Ile-Gln-Asn-Leu-Val-Pro-Glu-Asp-Lys-Leu
(c) Glu-Gln-Leu-Ile-Gln-Asn-Leu-Val-Pro-Glu-Asp-Lys-Leu-Ser
(d) Glu-Gln-Leu-Ile-Gln-Asn-Leu-Val-Pro-Glu-Asp-Lys-Leu-Ser-Arg
(e) Cys-Glu-Gln-Leu-Ile-Gln-Asn-Leu-Val-Pro-Glu-Asp-Lys
(f) Cys-Glu-Gln-Leu-Ile-Gln-Asn-Leu-Val-Pro-Glu-Asp-Lys-Leu
(g) Cys-Glu-Gln-Leu-Ile-Gln-Asn-Leu-Val-Pro-Glu-Asp-Lys-Leu-Ser
(h) Cys-Glu-Gln-Leu-Ile-Gln-Asn-Leu-Val-Pro-Glu-Asp-Lys-Leu-Ser-Arg
(i) Ile-Ser-Gly-Phe-Pro-Val-Gly-Arg,
(j) Ile-Ser-Gly-Phe-Pro-Val-Gly-Arg-Val,
(k) Gly-Ile-Ser-Gly-Phe-Pro-Val-Gly-Arg,
(l) Gly-Ile-Ser-Gly-Phe-Pro-Val-Gly-Arg-Val,
(m) Ser-Leu-Asn-Pro-Ala-Ile-Gly-Thr-Asp-Lys, and
(n) Cys-Ser-Leu-Asn-Pro-Ala-Ile-Gly-Thr-Asp-Lys,

**0 079 934**

wherein Gly represents glycine, and Glu, Gln, Leu, Ile, Asn, Val, Pro, Asp, Lys, Ser, Arg, Cys, Phe, Ala, and Thr, respectively represent the L-amino acid forms of glutamic acid, glutamine, leucine, isoleucine, asparagine, valine, proline, aspartic acid, lysine, serine, arginine, cysteine, phenylalanine, alanine, and threonine wherein synthesising of the linear sequences (a) to (n) is carried out by procedures known in the art.

9. A method according to claim 8, wherein the peptide compounds (a) to (h) include the 12 amino acid sequence of the compound (a).

10. A method according to claim 8, wherein the peptide compound (e) is prepared.

11. A method according to claim 8, wherein the peptide compounds (i) to (l) include the 8 amino acid sequence of the compound (i).

12. A method according to claim 8, wherein the peptide compound (l) is prepared.

13. A method according to claim 8, wherein the peptide compounds (m) and (n) are prepared.

14. A method according to claim 8, wherein the peptide compound (n) is prepared.

**Patentansprüche**

1. Peptidverbindungen, die in einer Sequenz von den N-terminalen zu den C-terminalen Aminosäuren angeordnet sind, ausgewählt aus der Klasse bestehend aus:

(a) Glu-Gln-Leu-Ile-Gln-Asn-Leu-Val-Pro-Glu-Asp-Lys
(b) Glu-Gln-Leu-Ile-Gln-Asn-Leu-Val-Pro-Glu-Asp-Lys-Leu
(c) Glu-Gln-Leu-Ile-Gln-Asn-Leu-Val-Pro-Glu-Asp-Lys-Leu-Ser
(d) Glu-Gln-Leu-Ile-Gln-Asn-Leu-Val-Pro-Glu-Asp-Lys-Leu-Ser-Arg
(e) Cys-Glu-Gln-Leu-Ile-Gln-Asn-Leu-Val-Pro-Glu-Asp-Lys
(f) Cys-Glu-Gln-Leu-Ile-Gln-Asn-Leu-Val-Pro-Glu-Asp-Lys-Leu
(g) Cys-Glu-Gln-Leu-Ile-Gln-Asn-Leu-Val-Pro-Glu-Asp-Lys-Leu-Ser
(h) Cys-Glu-Gln-Leu-Ile-Gln-Asn-Leu-Val-Pro-Glu-Asp-Lys-Leu-Ser-Arg
(i) Ile-Ser-Gly-Phe-Pro-Val-Gly-Arg,
(j) Ile-Ser-Gly-Phe-Pro-Val-Gly-Arg-Val,
(k) Gly-Ile-Ser-Gly-Phe-Pro-Val-Gly-Arg,
(l) Gly-Ile-Ser-Gly-Phe-Pro-Val-Gly-Arg-Val,
(m) Ser-Leu-Asn-Pro-Ala-Ile-Gly-Thr-Asp-Lys, and
(n) Cys-Ser-Leu-Asn-Pro-Ala-Ile-Gly-Thr-Asp-Lys,

wobei Gly Glycin repräsentiert und Glu, Gln, Leu, Ile, Asn, Val, Pro, Asp, Lys, Ser, Arg, Cys, Phe, Ala und Thr jeweils die L-Aminosäureform von Glutaminsäure, Glutamin, Leucin, Isoleucin, Asparagin, Valin, Prolin, Asparaginsäure, Lysin, Serin, Arginin, Cystein, Phenylalanin, Alanin und Threonin repräsentieren.

2. Peptidverbindungen (a) bis (h) des Anspruchs 1, die die aus zwölf Aminosäuren bestehende Sequenz aus der Verbindung (a) einschließen.

3. Peptidverbindung (e) nach Anspruch 1.

4. Peptidverbindungen (i) bis (l) nach Anspruch 1, die die aus acht Aminosäuren bestehende Sequenz aus der Verbindung (i) einschließen.

5. Peptidverbindung (l) nach Anspruch 1.

6. Peptidverbindungen (m) und (n) nach Anspruch 1.

7. Peptidverbindung (n) nach Anspruch 1.

8. Verfahren zum Herstellen von antigenen Peptidverbindungen, die in einer Sequenz von den N-terminalen zu den C-terminalen Aminosäuren angeordnet sind, ausgewählt aus der Klasse bestehend aus:

(a) Glu-Gln-Leu-Ile-Gln-Asn-Leu-Val-Pro-Glu-Asp-Lys
(b) Glu-Gln-Leu-Ile-Gln-Asn-Leu-Val-Pro-Glu-Asp-Lys-Leu
(c) Glu-Gln-Leu-Ile-Gln-Asn-Leu-Val-Pro-Glu-Asp-Lys-Leu-Ser
(d) Glu-Gln-Leu-Ile-Gln-Asn-Leu-Val-Pro-Glu-Asp-Lys-Leu-Ser-Arg
(e) Cys-Glu-Gln-Leu-Ile-Gln-Asn-Leu-Val-Pro-Glu-Asp-Lys
(f) Cys-Glu-Gln-Leu-Ile-Gln-Asn-Leu-Val-Pro-Glu-Asp-Lys-Leu
(g) Cys-Glu-Gln-Leu-Ile-Gln-Asn-Leu-Val-Pro-Glu-Asp-Lys-Leu-Ser
(h) Cys-Glu-Gln-Leu-Ile-Gln-Asn-Leu-Val-Pro-Glu-Asp-Lys-Leu-Ser-Arg
(i) Ile-Ser-Gly-Phe-Pro-Val-Gly-Arg,
(j) Ile-Ser-Gly-Phe-Pro-Val-Gly-Arg-Val,
(k) Gly-Ile-Ser-Gly-Phe-Pro-Val-Gly-Arg,
(l) Gly-Ile-Ser-Gly-Phe-Pro-Val-Gly-Arg-Val,
(m) Ser-Leu-Asn-Pro-Ala-Ile-Gly-Thr-Asp-Lys, and
(n) Cys-Ser-Leu-Asn-Pro-Ala-Ile-Gly-Thr-Asp-Lys,

wobei Gly Glycin repräsentiert und Glu, Gln, Leu, Ile, Asn, Val, Pro, Asp, Lys, Ser, Arg, Cys, Phe, Ala und Thr

**0 079 934**

jeweils die L-Aminosäureform von Glutaminsäure, Glutamin, Leucin, Isoleucin, Asparagin, Valin, Prolin, Asparaginsäure, Lysin, Serin, Arginin, Cystein, Phenylalanin, Alanin und Threonin repräsentieren.

9. Verfahren zum Herstellen der Peptidverbindungen (a) bis (h) des Anspruchs 8, die die aus zwölf Aminosäuren bestehende Sequenz aus der Verbindung (a) einschließen.

10. Verfahren zum Herstellen der Peptidverbindung (e) nach Anspruch 8.

11. Verfahren zum Herstellen der Peptidverbindungen (i) bis (l) nach Anspruch 8, die die aus acht Aminosäuren bestehende Sequenz der Verbindung (i) einschließen.

12. Verfahren zum Herstellen der Peptidverbindung (l) nach Anspruch 8.

13. Verfahren zum Herstellen der Peptidverbindung (m) und (n) nach Anspruch 8.

14. Verfahren zum Herstellen der Peptidverbindung (n) nach Anspruch 8.


## Revendications

1. Peptides antigènes disposés dans une séquence d'amino-acides N-Terminal à C-Terminal choisis dans la classe constituée de:

    (a) Glu-Gln-Leu-Ile-Gln-Asn-Leu-Val-Pro-Glu-Asp-Lys
    (b) Glu-Gln-Leu-Ile-Gln-Asn-Leu-Val-Pro-Glu-Asp-Lys-Leu
    (c) Glu-Gln-Leu-Ile-Gln-Asn-Leu-Val-Pro-Glu-Asp-Lys-Leu-Ser
    (d) Glu-Gln-Leu-Ile-Gln-Asn-Leu-Val-Pro-Glu-Asp-Lys-Leu-Ser-Arg
    (e) Cys-Glu-Gln-Leu-Ile-Gln-Asn-Leu-Val-Pro-Glu-Asp-Lys
    (f) Cys-Glu-Gln-Leu-Ile-Gln-Asn-Leu-Val-Pro-Glu-Asp-Lys-Leu
    (g) Cys-Glu-Gln-Leu-Ile-Gln-Asn-Leu-Val-Pro-Glu-Asp-Lys-Leu-Ser
    (h) Cys-Glu-Gln-Leu-Ile-Gln-Asn-Leu-Val-Pro-Glu-Asp-Lys-Leu-Ser-Arg
    (i) Ile-Ser-Gly-Phe-Pro-Val-Gly-Arg,
    (j) Ile-Ser-Gly-Phe-Pro-Val-Gly-Arg-Val,
    (k) Gly-Ile-Ser-Gly-Phe-Pro-Val-Gly-Arg,
    (l) Gly-Ile-Ser-Gly-Phe-Pro-Val-Gly-Arg-Val,
    (m) Ser-Leu-Asn-Pro-Ala-Ile-Gly-Thr-Asp-Lys, et
    (n) Cys-Ser-Leu-Asn-Pro-Ala-Ile-Gly-Thr-Asp-Lys,

dans laquelle Gly représente la glycine et Glu, Gln, Leu, Ile, Asn, Val, Pro, Asp, Lys, Ser, Arg, Cys, Phe, Ala et Thr, représentent respectivement les formes L-amino-acides de l'acide glutamique, de la glutamine, de la leucine, de l'isoleucine, de l'asparagine, de la valine, de la proline, de l'acide aspartique, de la lysine, de la sérine, de l'arginine, de la cystéine, de la phénylalanine, de l'alanine et de la thréonine.

2. Peptides a à h de la revendication 1, qui comprennent la séquence de 12 amino-acides de a.

3. Peptides e de la revendication 1.

4. Peptides i à l de la revendication 1, qui comprennent la séquence de 8 amino-acides de i.

5. Peptide l de la revendication 1.

6. Peptides m et n de la revendication 1.

7. Peptide n de la revendication 1.

8. Procédé de préparation de composés peptides antigéniques disposés dans une séquence d'amino-acides N-terminal à C-terminal choisis dans la class constituée de:

    (a) Glu-Gln-Leu-Ile-Gln-Asn-Leu-Val-Pro-Glu-Asp-Lys
    (b) Glu-Gln-Leu-Ile-Gln-Asn-Leu-Val-Pro-Glu-Asp-Lys-Leu
    (c) Glu-Gln-Leu-Ile-Gln-Asn-Leu-Val-Pro-Glu-Asp-Lys-Leu-Ser
    (d) Glu-Gln-Leu-Ile-Gln-Asn-Leu-Val-Pro-Glu-Asp-Lys-Leu-Ser-Arg
    (e) Cys-Glu-Gln-Leu-Ile-Gln-Asn-Leu-Val-Pro-Glu-Asp-Lys
    (f) Cys-Glu-Gln-Leu-Ile-Gln-Asn-Leu-Val-Pro-Glu-Asp-Lys-Leu
    (g) Cys-Glu-Gln-Leu-Ile-Gln-Asn-Leu-Val-Pro-Glu-Asp-Lys-Leu-Ser
    (h) Cys-Glu-Gln-Leu-Ile-Gln-Asn-Leu-Val-Pro-Glu-Asp-Lys-Leu-Ser-Arg
    (i) Ile-Ser-Gly-Phe-Pro-Val-Gly-Arg,
    (j) Ile-Ser-Gly-Phe-Pro-Val-Gly-Arg-Val,
    (k) Gly-Ile-Ser-Gly-Phe-Pro-Val-Gly-Arg,
    (l) Gly-Ile-Ser-Gly-Phe-Pro-Val-Gly-Arg-Val,
    (m) Ser-Leu-Asn-Pro-Ala-Ile-Gly-Thr-Asp-Lys, et
    (n) Cys-Ser-Leu-Asn-Pro-Ala-Ile-Gly-Thr-Asp-Lys,

dans laquelle Gly représente la glycine et Glu, Gln, Leu, Ile, Asn, Val, Pro, Asp, Lys, Ser, Arg, Cys, Phe, Ala et Thr, représentent respectivement les formes L-amino-acides de l'acide glutamique, de la glutamine, de la leucine, de l'isoleucine, de l'asparagine, de la valine, de la proline, de l'acide aspartique, de la lysine, de la sérine, de l'arginine, de la cystéine, de la phénylalanine, de l'alanine et de la thréonine, dans lequel la synthèse des séquences linéaires a à n est effectuée selon des procédés connus dans la technique.

9. Procédé selon la revendication 8, dans lequel les composés peptides a à h comprennent la séquence de 12 amino-acides du composé a.

10. Procédé selon la revendication 8, dans lequel le composé peptide e est préparé.

11. Procédé selon la revendication 8, dans lequel les composés peptides i à l comprennent la séquence de 8 amino-acides du composé i.

12. Procédé selon la revendication 8, dans lequel le composé peptide l est préparé.

13. Procédé selon la revendication 8, dans lequel les composés peptides m et n sont préparés.

14. Procédé selon la revendication 8, dans lequel le composé peptide n est préparé.